# EUROPEAN PATENT APPLICATION

(11) **EP 0 636 353 A1**
(43) Date of publication of application: **01.02.1995**
(21) Application number: 94830386.2
(22) Date of filing: 28.07.1994
(51) Int. Cl.: A61F 2/38

(54) **Full knee prosthesis**

(30) Priority: 28.07.1993 IT MI931688
(71) Applicant: G. CREMASCOLI S.R.L., I-20138 Milano (IT)
(72) Inventor: Cremascoli, Patrizio, c/o G. Cremascoli S.p.A., I-20138 Milano (IT)
(74) Representative: Cicogna, Franco

(57) **Abstract**

The prosthesis comprises a femural component to be connected to the distal portion of the femur and comprising two curved portions operating as a medial condyle and a side condyle,a tibial component to be connected to the proximal end of the tibia, with a flat top surface,and a tibial insert which can be engaged on the top surface to be movable with respect to the tibial component,the tibial insert being provided at the top thereof with two concave surfaces which can be engaged by the two portions of the femural component.

## Description

### BACKGROUND OF THE INVENTION

The present invention relates to a full knee prosthesis.

As is known,great problems are involved in making full knee prostheses,since has not been heretofore possible to provide prostheses adapted to precisely reproduce the main knee displacements,that is the rolling and rear sliding of the femural condyles on the tibia plate, with the possibility of allowing the tibia to rotate with respect to the mentioned femural condyles.

Thus,prior full knee prostheses have not been practically broadly used,because of the great drawbacks brought to the deambulation.

### SUMMARY OF THE INVENTION

Accordingly,the aim of the present invention is to solve the above mentioned problem,by providing a full knee prosthesis which allows to reproduce the main functions of the knee articulation, during the knee bending operations and, more specifically, the rolling and rear sliding of the femural condyles,on the tibial plate portion, while allowing the tibia to rotate with respect to the femural condyles.

Within the above aim,a main object of the present invention is to provide such a full knee prosthesis the component of which are so designed as to reduce to a minimum the wear thereof, owing to a perfect mutual fitting of all the articulation surfaces in all the assumed positions.

Another object of the invention is to provide such a full knee prosthesis which affords a better distribution of the applied forces,so as to prevent localized stresses from occurring which would quickly deteriorate the materials of the prosthesis components with a consequent reduction of the prosthesis performance and safety.

According to one aspect of the present invention,the above mentioned aim and objects,as well as yet other objects,which will become more apparent hereinafter,are achieved by a full knee prosthesis, characterized in that said prosthesis comprises: a femural component,which can be connected to the distal end of the femur and including two curved portions having the functions of medial condyle and side condyle,a tibial component,which can be coupled to the proximal end of the tibia,with a flat top surface,and a tibial insert which can be engaged on the top surface so as to be movable with respect to the tibial component,the tibial insert being provided at the top thereof with two concave surfaces which can be engaged by the two portions of the femur components.

### BRIEF DESCRIPTION OF THE DRAWINGS

Further characteristics and advantages of the present invention will become more apparent hereinafter from the following detailed disclosure of a preferred though not exclusive,embodiment of a full knee prosthesis which is illustrated by way of an indicative,but not limitative example,in the fires of the accompanying drawings where:
Figure 1 is an exploded perspective view illustrating schematically the prosthesis according to the invention;
Figure 2 is a perspective view illustrating the tibial insert;
Figure 3 is a further perspective view illustrating an element to be possibly connected to the tibial component in order to improve the anchoring thereof;

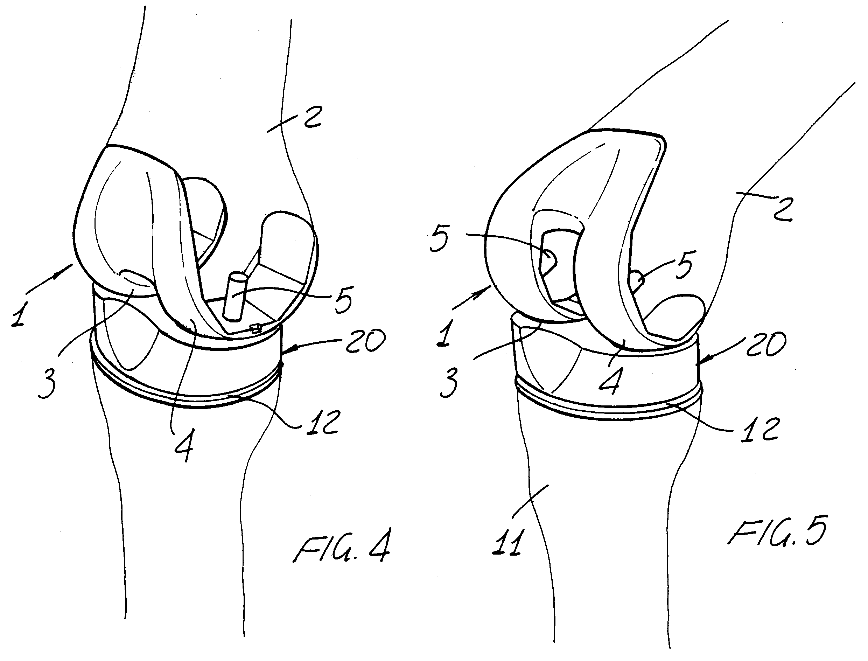

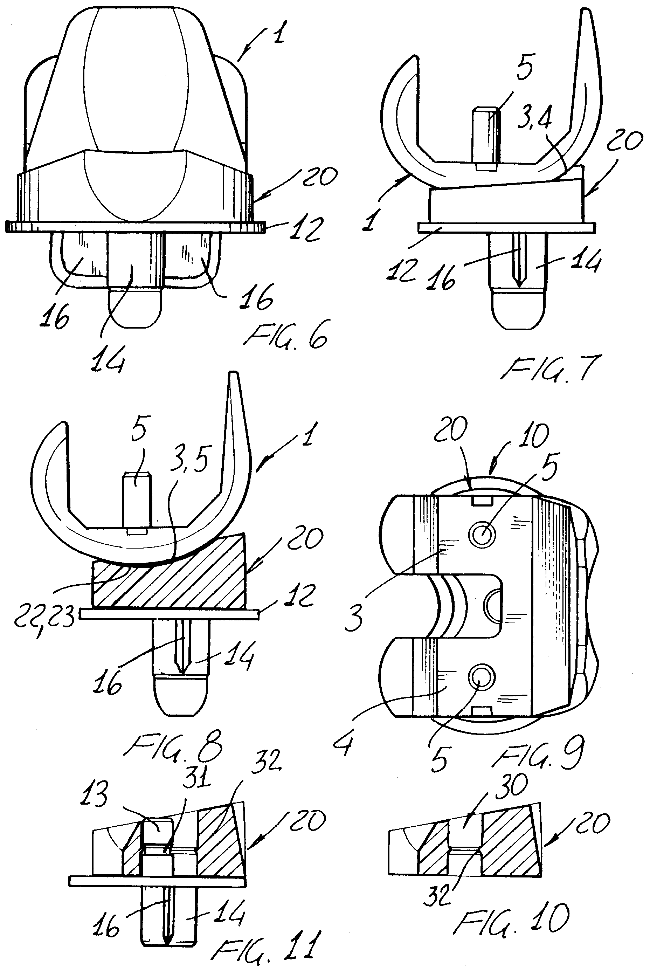

Figures 4 and 5 are further perspective views illustrating the prosthesis applied to the femural and tibial bones,at two different mutual positions;
Figure 6 is a schematic elevation view taken from the front of the prosthesis according to the present invention;
Figure 7 is a side elevation view of the prosthesis;
Figure 8 is a further side elevation view of the prosthesis with the tibial insert being shown in cross-section;
Figure 9 is a top plan view of the prosthesis;
Figures 10 and 11 represent the tibial insert,respectively,with a circular hole and an elongated hole,the latter being engaged with the tibial component;
Figures 12,13 and 14 represent,schematically,several mutual positions of the tibial insert and tibial components,which elements can be mutually rotated or turned;
   and
Figures 15,16 and 17 represent the tibial insert and tibial component at several mutual positions with a connection allowing a rotary translating movement thereof.

### DESCRIPTION OF THE PREFERRED EMBODIMENTS

With reference to the number references of the figures of the accompanying drawings,the full knee prosthesis according to the present invention, comprises a femural component generally indicated at the reference number 1,which can be connected to the distal portion of the femur 2.

The femural component 1 is provided with two portions 3 and 4 which have a curved configuration and are adapted to reproduce the functions of medial condyle and side condyle.

The outer surfaces of the portions 3 and 4 have been specifically designed to provide a precise fitting with the other components of the prosthesis.

On the inner surfaces of the portions 3 and 4,which form the condyle portions,there are provided pins,both indicated at the reference number 5, having a cylindrical shape and preventing any mutal movements between the femural component 1 and the femur 2.

The pins 5,in particular,can be made in single piece with the femural component 1 or,if desired,they can be connected with a forced type of fitting or connection.

The prosthesis comprises moreover a tibial component,which has been generally indicated at the reference number 10,which will be connected to the proximal end portion of the tibia 11.

This tibial component has,shown from the top,a substantially horseshoe configuration.

The top face 12 is perfectly flat and is polished by an end mirror-like polishing process.

On the central portion of the top surface 12 there is provided a projecting cylindric pin 13 on the upper or top portion of which there is provided a groove 31.

The pin 13 cooperates with the tibial insert 20 as it will be disclosed hereinafter.

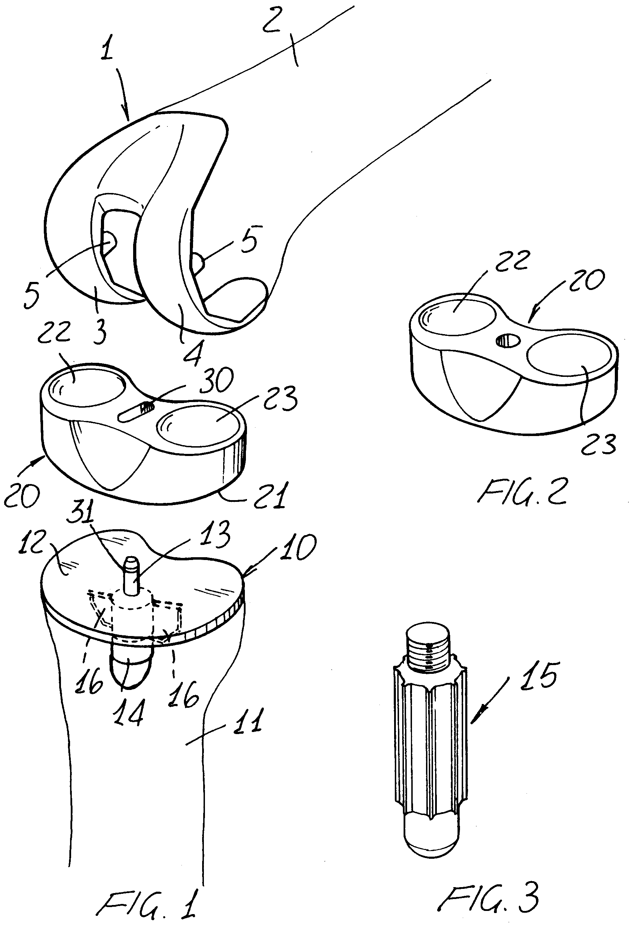

From the bottom portion of the tibial component there extends a cylindric bush 14 which is provided with a threaded axial hole,in which a plug element or stabilizing element 15,which is better shown in figure 3,can be threaded.

This stabilizing element,in particular,can be used for allowing a further anchoring of the tibial component.

From the cylindric bush 14 there extend two radial legs 16 adapted to improve the connection of the tibial component to the tibia,thereby preventing any mutual rotation about the axis of the bush.

Differently from the top surface,the bottom surface of the tibial component 10 is very rough.

It is in fact subjected to a sanding process to allow a perfect integrating coupling with the bone material.

Between the femural component 1 and the tibial component 10 there is arranged a tibial insert 20 which cooperates with the pin 13.

The bottom face 21 of the tibial insert 20 is perfectly flat and smooth,whereas,on the top part thereof there are provided two concave surfaces,indicated at 22 and 23 which are coupled with the portions 3 and 4 forming the condyle regions.

Through the central portion of the insert there is provided a throughgoing slot 30 the cross section of which becomes narrower at the middle region because of the provision of a ridge element 32.

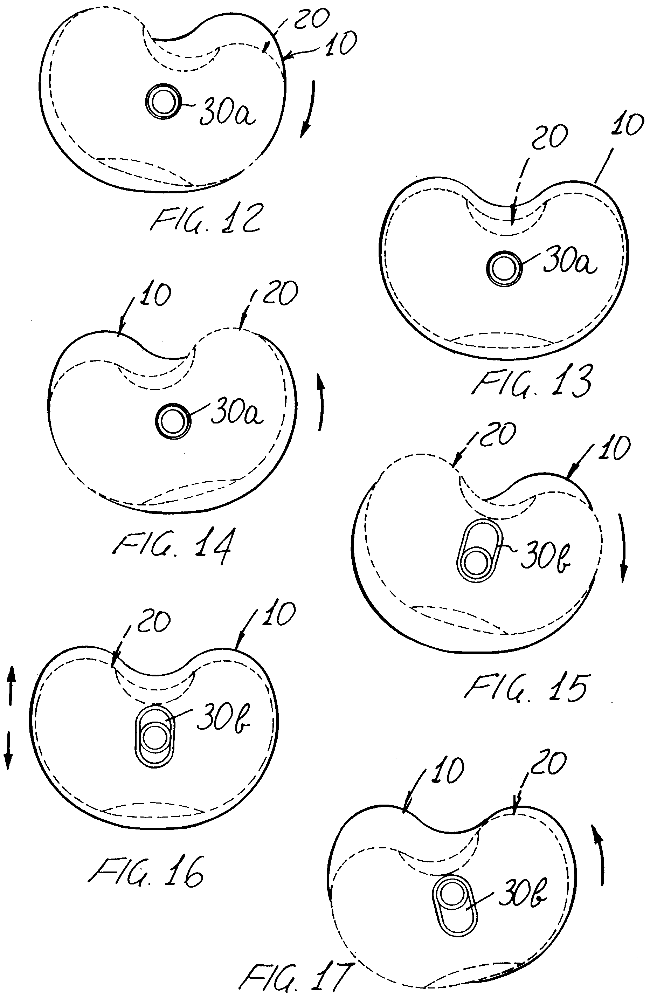

The slot 30 can have a circular shape indicated at 30a in figures 12 to 14,or an elongated shape indicated at 30b in figures 15 to 17.

In said slot 30 there is engaged the pin 13 of the tibial component.

The cooperation between the groove 31 of the pin 13 and the ridge 32 of the tibial insert 20 will prevent said tibial insert from disengaging in the axial direction.

The cooperation between the pin 13 and tibial insert 20 will prevent,moreover,possible dislocations of said tibial insert,both in a front-rear direction and in a side-medial direction.

With the disclosed arrangement,the tibial insert can turn,if the circular slot 30a is used,on the tibial metal plate;this solution will be used in those cases in which,during an operation,will be sacrificed both the rear crossed ligament and the front crossed ligament.

The insert can rotate and translate on the metal tibial plate by using the slot 30b.

This solution will be used for those cases in which,during the operation,is sacrficed only the front crossed ligament while the rear cross ligament is not affected.

The portions 3 and 4 are designed,as stated, to perfectly fit or mate with the concave surfaces of the tibial insert,so as to provide a perfect articulation.

Moreover,the tibial component and tibial insert are so designed that it will be possible to assemble a tibial insert of one or more size less than the tibial component on which the tibial insert will be mounted.

With the disclosed arrangement,the subject prosthesis will reproduce as faithfully as possible all of the physiologic movements of the articulation, and,furthermore,the used materials will be subjected to a very small wear.

The physiologic movements of the articulation in particular are preserved since in the embodiment using a tibial insert having a circular shape slot 30,there is held both the rotary movement of the femural component on the tibia plate and the rotary possibility of the tibia.

Does not intervene the rear sliding of the femural condyles since in this case there are sacrificed both the crossed ligaments and,under such a condition,the rear sliding does not occur.

In the embodiment in which there are provided a rotation and a translation,as used in those cases in which is preserved intact the rear crossed ligament, one has also the possibility of preserving the rear sliding.

Moreover,the wear of the components is practically eliminated owing to a perfect mating of the articulation surfaces,at any mutual positions assumed by the components.

As known,a perfect surface mating will involve a better distribution of the forces,preventing localized stresses from occurring,which could cause a wear and reduction of the functionality of the prosthesis.

The invention,as disclosed,is susceptible to several modifications and variations all of which will come within the scope of the invention.

Moreover,all of the details can be replaced by other technically equivalent elements.

In practicing the invention,the used material can be any even if from experiments it has been found that it is very convenient to use a femur component and a tibial component made of a Cr-Co-Mo alloy with a possible coating of the surface contacting the bone by hydroxyapatite.

The stabilizing element and plug being made of a titanium alloy or a Cr-Co-Mo alloy.

The tibial insert is made of polyethylene having a vary high molecular weight.

## Claims

1. A full knee prosthesis characterized in that said prosthesis comprises:a femural component,which can be connected to the distal end of the femur and including two curved portions having the functions of a medial condyle and a side condyle,a tibial component,which can be coupled to the proximal end of the tibia,with a flat top surface and a tibial insert which can be engaged on the top surface so as to be movable with respect to the tibial component,the tibial insert being provided at the top thereof with two concave surfaces which can be engaged by the two portions of the femur components.

2. A prosthesis according to claim 1,characterized in that said femural component has convex outer surfaces precisely mating with the concave surfaces of the tibial insert.

3. A prosthesis according to claims 1 and 2,characterized in that said prosthesis further comprises cylindric pins extending from the inner surface of the two portions.

4. A prosthesis according to one or more of the preceding claims, characterized in that said tibial component has,seen from the top,a substantially horseshoe configuration.

5. A prosthesis according to one or more of the preceding claims,characterized in that the top surface of the tibial component is polished by an end mirror-like polishing process.

6. A prosthesis according to one or more of the preceding claims,characterized in that said prosthesis further comprises a pin extending from a middle portion of the tibial component and engaging in a throughgoing slot provided through the tibial insert.

7. A prosthesis according to one or more preceding claims,characterized in that said prosthesis further comprises a cylindric bush extending from the bottom face of the tibial component and that in said bush there is provided an axial threaded hole for screw engaging therein a plug or stabilizing element.

8. A prosthesis according to one or more of the precding claims,characterized in that said prosthesis further comprises radial legs extending from said cylindric bush.

9. A prosthesis according to one or more of the preceding claims,characterized in that the bottom surface of the tibial component is rough so as to allow a bone integration.

10. A prosthesis according to one or more of the preceding claims,characterized in that said tibial insert is provided,between the two concave surfaces, with a slot which can be engaged by the pin of the tibial component.

11. A prosthesis according to one or more of the preceding claims,characterized in that said slot has a substantially circular shape.

12. A prosthesis according to one or more of the preceding claims,characterized in that said slot has an elongated shape.

13. A prosthesis according to one or more of the preceding claims,characterized in that,as said slot has an elongated shape,said tibial insert can be both rotated and translated with respect to said tibial component.

14. Aprosthesis according to one or more of the preceding claims,characterized in that said prosthesis is moreover provided with an insert through a central portion of which there is provided a throughgoing slot the cross section of which narrows at the middle region because of the provision of a ridge,said slot having either a circular shape or an elongated shape.

15. A prosthesis according to one or more of the preceding claims,characterized in that in said slot there is engaged the pin of the tibial component,whereas between the groove,the pin and the ridge of the tibial insert there occurs a cooperation preventing said tibial insert from disengaging in the axial direction,the cooperation between the pin and tibial insert preventing said tibial insert from being dislocated both in a front-rear direction and in a side-medial direction.

16. A prosthesis according to one or more of the preceding claims,characterized in that from the central portion of the top surface there extends a cylindric pin in the top region of which there is formed a groove to allow said pin to cooperate with said tibial insert.
